# EUROPÄISCHE PATENTSCHRIFT

(11) **EP 3 257 544 B1**
(45) Veröffentlichungstag und Bekanntmachung des Hinweises auf die Patenterteilung: **18.09.2019**
(21) Anmeldenummer: 17001001.1
(22) Anmeldetag: 13.06.2017
(51) Int. Cl.: A61M 16/20, A61M 16/08

(54) **NOTFALLAUSATEMVENTIL MIT MEMBRANRING**
EMERGENCY BREATHING VALVE WITH MEMBRANE RING
SOUPAPE D'EXPIRATION DE SECOURS COMPRENANT UNE BAGUE DE MEMBRANE

(30) Priorität: 14.06.2016 DE 102016007202
(43) Veröffentlichungstag der Anmeldung: 20.12.2017
(73) Patentinhaber: Löwenstein Medical Technology S.A., 2557 Luxembourg (LU)
(72) Erfinder: Gardein, Joachim, 38430 Icod de los Vinos Tenerife (ES)
(74) Vertreter: Marx, Thomas

(56) Entgegenhaltungen:
- EP-A1- 2 845 618
- WO-A1-02/051486
- WO-A1-2007/045008
- WO-A1-2013/006899
- WO-A1-2013/171705
- DE-U1-202011 107 053
- US-A1- 2004 255 948
- US-A1- 2009 272 380
- US-A1- 2010 258 133
- US-A1- 2016 015 918

## Beschreibung

### Gebiet der Erfindung

Die vorliegende Erfindung betrifft eine als Patienteninterface ausgebildete Beatmungsvorrichtung mit einem integrierten Notfallausatemventil. Beatmungsvorrichtungen sind für Patienten von Bedeutung, die unter einer Störung der Atemluft- oder Sauerstoffzufuhr leiden, wobei dem Patienten über ein Patienteninterface oft als Vollgesichtsmaske ausgebildet, mit einer positiven Druckunterstützung Atemgas oder mit Sauerstoff angereicherte Luft zugeführt wird.

### Stand der Technik

Notfallausatemventile (AAV - Anti-Asphyxie-Ventile) sind aus dem Stand der Technik bekannt. Sie dienen dem Patienten dazu im Falle einer Unterbrechung der Beatmung beispielsweise bei Störung oder Ausfall des Therapiegerätes über eine Atmosphärenöffnung Raumluft einzuatmen und die CO₂-angereicherte Ausatemluft an die Umgebung abzugeben.

Das Dokument WO 2013/171705 A1 offenbart ein Anti-Asphyxie-Ventil mit einem Membranelement, das eine Vielzahl von blütenblattförmigen Dichtungselementen, die sich von einem Basisabschnitt nach oben erstrecken. Dabei weisen die Dichtungselemente variierende Querschnittsdicken auf.

Das Dokument US 2016/015918 A1 betrifft ein System zum Liefern von Atemgasen an einen Benutzer, wobei das System zwischen einem ersten Druck und einem zweiten Druck synchron mit einem Atemzustand des Benutzers schaltet. Das System umfasst eine Strömungsumlenkvorrichtung, die in einem zentralen Bereich eine Ummantelung aufweist, die eine Öffnung zum Ausströmen von Atemgas definiert.

Das Dokument WO 2007/045008 A1 betrifft eine Ellbogenanordnung für eine Maskenanordnung, die eine Anti-Asphyxie-Ventil (AAV) - Anordnung umfasst. Die AAV-Anordnung kann ein Klappenelement umfassen, das bewegbar ist. Die Ellenbogenanordnung kann zudem einen Schlitz aufweisen, um die AAV-Anordnung aufzunehmen und ein Clipelement, dass an einer Außenfläche des Ellbogens befestigt ist.

### Kritik am Stand der Technik

Die aus dem Stand der Technik bekannten Anti-Asphyxie-Ventile erfüllen nicht alle Anforderungen an Sicherheit und Hygiene bzw. sind teuer und/oder aufwendig in der Herstellung.

### Erfindung

Ausgehend vom Stand der Technik liegt der Erfindung die Aufgabe zu Grunde, ein verbessertes Anti-Asphyxie-Ventil zu realisieren.

Die Aufgabe wird durch einen Anschlussstutzen für die Beatmung gelöst, mit einer Öffnung die zum Beatmungsschlauch weist und einer Öffnung die zum Patienteninterface angeordnet ist und einer Atmosphärenöffnung die von innen durch eine Membran zumindest teilweise verschließbar ist, wobei der Anschlussstutzen eine seitliche Membranöffnung, für die Aufnahme der Membran in den Stutzen, aufweist und die Membran über einen Membranring, der den Anschlussstutzen in vollem Umfang umschließt, gehalten ist.

Die Erfindung ist ergänzend dadurch gekennzeichnet, dass der Anschlussstutzen im Außenumfang zumindest abschnittsweise eine Nut für den Membranring aufweist.

Die Erfindung ist auch dadurch gekennzeichnet, dass ein Führungssteg am Membranring sowie eine korrespondierende Führungsaufnahme im Anschlussstutzen die. Einbauposition von Membranring und Membran festlegen.

Die Erfindung kann auch dadurch gekennzeichnet sein, dass der Membranweg im Anschlussstutzen durch zumindest einen Anschlag und zumindest einen Steg begrenzt ist und Anschlag und Steg jeweils eine Anlagefläche für die Membran bilden.

Die Erfindung ist beispielsweise auch dadurch gekennzeichnet, dass der Anschlussstutzen verbindungselemente zur Verbindung mit einer Drehhülse aufweist.

Die Erfindung ist alternativ auch dadurch gekennzeichnet, dass der Anschlussstutzen zumindest abschnittsweise einen Außendurchmesser aufweist, der dem Außendurchmesser der Drehhülse, zumindest abschnittsweise, entspricht.

Die Erfindung ist auch dadurch gekennzeichnet, dass der Anschlussstutzen zumindest abschnittsweise einen Außendurchmesser aufweist, der dem Außendurchmesser des Membranringes entspricht.

Die Erfindung ist beispielsweise auch dadurch gekennzeichnet, dass der Anschlussstutzen zumindest im Bereich der Membranöffnung einen Außendurchmesser aufweist, der größer ist als der Innendurchmesser des Membranringes.

Die Erfindung ist beispielsweise auch dadurch gekennzeichnet, dass die Breite des Ringes ist zumindest abschnittsweise größer ist als die Breite der Nut.

Die Erfindung ist beispielsweise auch dadurch gekennzeichnet, dass die Membranöffnung konisch nach innen verjüngend ausgeführt ist.

Die Erfindung ist beispielsweise auch dadurch gekennzeichnet, dass die Membran ist über ein Scharnier mit dem Membranring verbunden ist.

Die Erfindung ist beispielsweise auch dadurch gekennzeichnet, dass zwischen dem Scharnier und dem Ring noch ein Dichtbereich angeordnet ist, der zum Verschließen der Membranöffnung im Anschlussstutzen dient.

Die Erfindung ist beispielsweise auch dadurch gekennzeichnet, dass der Membranring mit der Membran einteilig aus Silikon oder einem anderen Elastomer gefertigt ist.

Die Erfindung ist beispielsweise auch dadurch gekennzeichnet, dass die Membran eine Stärke bevorzugt von 0,4 mm bis 1,0 mm aufweist und das Scharnier dünner ist als die Membran.

Die Erfindung ist beispielsweise auch dadurch gekennzeichnet, dass zwischen der Membran und dem Innendurchmesser des Ringes ein Spalt verbleibt, um ein freies Schwingen der Membran zu ermöglichen.

Die folgenden Figuren zeigen die Anordnung des Notfallausatemventils.
- Fig. 1:: Anschlussstutzen mit Atmosphärenöffnung und Drehhülse
- Fig. 2:: Explosionszeichnung mit Membranring
- Fig. 3:: Schnittdarstellung mit eingesetzter Ringmembran
- Fig. 4:: verschiedene Ansichten des Anschlussstutzens
- Fig. 5:: verschiedene Ansichten der Ringmembran

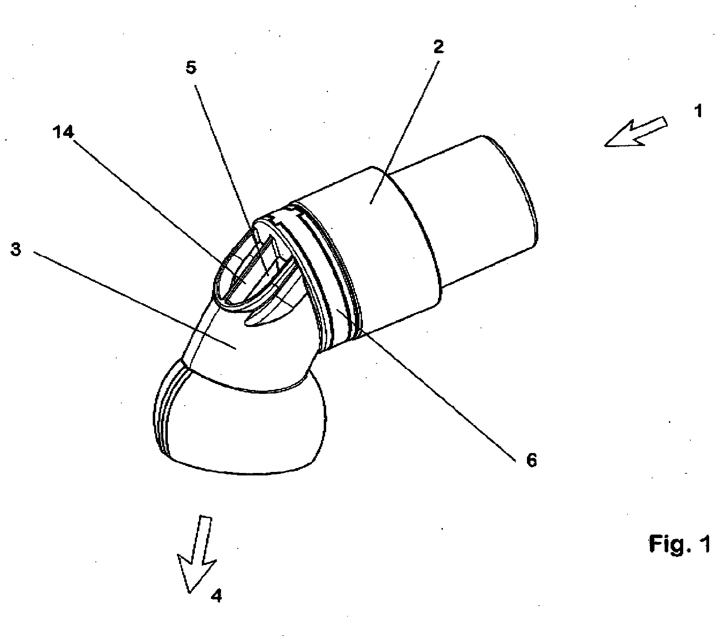

Fig. 1 zeigt einen Anschlussstutzen (3) für die Beatmung mit einer Öffnung die zum Beatmungsschlauch (1) weist und einer Öffnung die zum Patienteninterface (4) angeordnet ist und einer Atmosphärenöffnung (5) die von innen durch eine Membran (7) zumindest teilweise verschließbar ist, wobei der Anschlussstutzen (3) eine Membranöffnung (13), für die Aufnahme der Membran (7) in den Stutzen, aufweist und die Membran (7) über einen Membranring (6), der den Anschlussstutzen (3) umschließt, gehalten ist.

Das Therapiegerät wird über einen Beatmungsschlauch (1), eine Drehhülse (2) und einen Anschlussstutzen (3), beispielsweise als winkelanschluss mit Kugelgelenk ausgebildet, mit dem Patienteninterface (4), beispielsweise einer Beatmungsmaske, verbunden und dient der Zufuhr von Atemgas zum Patienten. Der Anschlussstutzen (3) ist luftleitend zwischen Schlauch (1) und Patienteninterface (4) angeordnet. Im Anschlussstutzen (3) ist eine Atmosphärenöffnung (5) angeordnet, um eine ungehinderte Zu- bzw. Ableitung der Luft bei Therapieunterbrechungen zu ermöglichen. In den Flowkanal (11) (hier als Pfeil dargestellt) ist ein Membranring (6) so eingesetzt, dass die Membran (7) die Atmosphärenöffnung (5) im normalen Therapiebetrieb durch den anliegenden Druck verschließt. Der Therapiebetrieb lenkt dabei die Membran aus ihrer Ausgangslage aus. Bei einem Druckabfall klappt die Membran in ihre Ausgangslage zurück. Dadurch verschließt die Membran in Richtung zum Schlauch (1) zumindest teilweise und verhindert so eine Rückatmung in den Beatmungsschlauch (1). Der Patient kann jetzt Umgebungsluft über die Atmosphärenöffnung (5) atmen. Figur 1 zeigt perspektivisch den Aufbau des Anschlussstutzens mit der Atmosphärenöffnung, dem eingesetzten Membranring (6) und einer Drehhülse, aber ohne den Schlauch und die Beatmungsmaske.

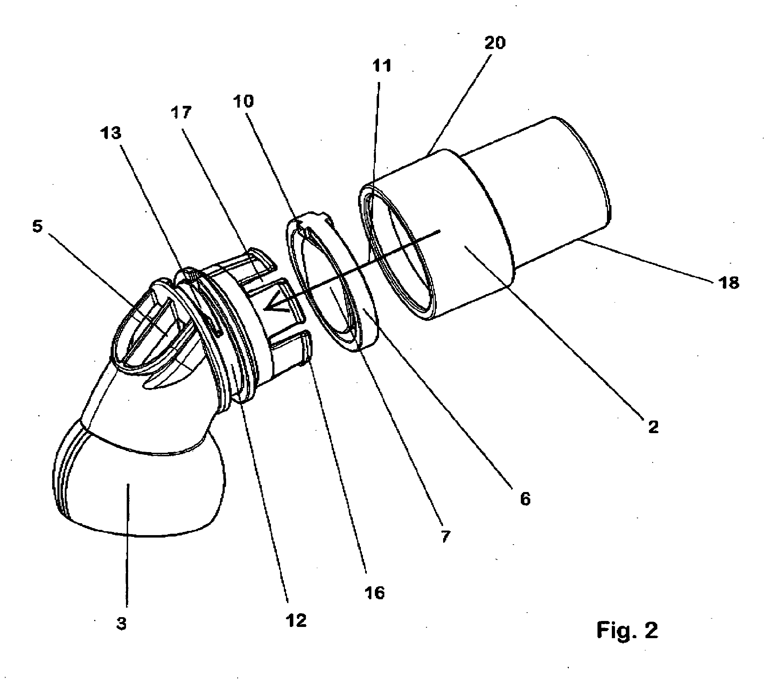

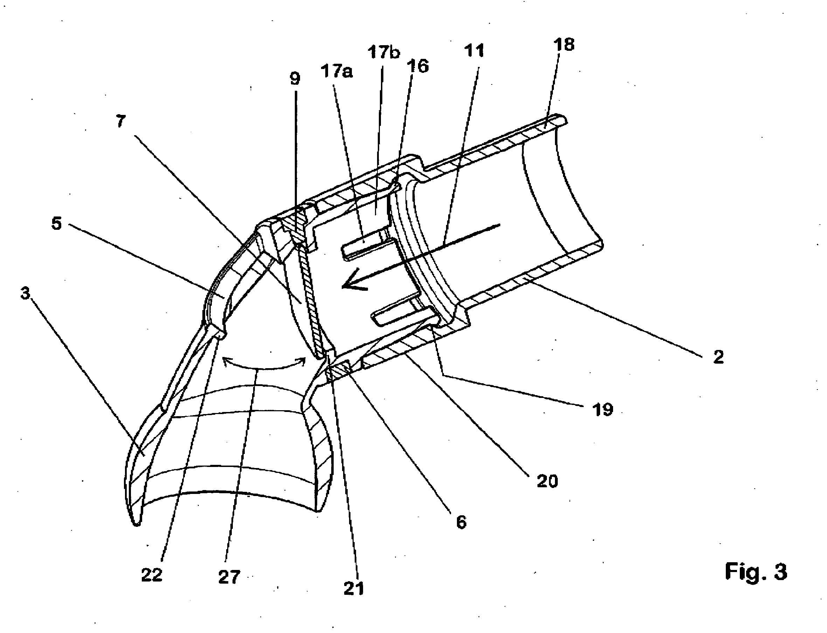

Fig.2 zeigt den gleichen Aufbau in einer Explosionsdaratellung und Fig.3 in einer Schnittdarstellung.

Der Anschlussstutzen (3), wie in Fig.2 dargestellt, weist eine Membranöffnung (13) und beispielsweise eine Nut (12) im Außenumfang auf. Die Nut (12) kann sowohl umlaufend als auch nur in Teilbereichen am Umfang des Anschlussstutzens (3) angeordnet sein und dient zur Aufnahme und Fixierung des Membranringes (6). Der Membranring ist beispielsweise einteilig aus silikon oder einem anderen Elastomer gefertigt. Die Membran (7), die sich an dem Membranring (6) befindet, wird durch die Membranöffnung (13) in den Flowkanal (11) ins Innere des Anschlussstutzens (3) eingeführt. Der Ring (6a) des Membranringes (6) wird dabei über die Schlauchanschlussseite gezogen bis er sich in der Nut (12) fixieren lässt. Dazu ist die Drehhülse (2) demontiert. Ein Führungssteg (10) am Membranring (6) sowie die korrespondierende Führungsaufnahme (28) im Anschlussstutzen. (3) dienen der Verdrehsicherung und gewährleisten eine korrekte Einbauposition der Membran (7).

Die Schnittdarstellung Figur 3 zeigt die Lage der Membran während einer Therapiestörung. Die Membran (7) des Notfallausatemventil verschließt den Flowkanal und gibt somit die Atmosphärenöffnung (5) frei. Der Membranweg (27) ist hier mit dem gebogenen Pfeil dargestellt und wird beispielsweise durch je eine Anschlag (21, 22) und einen Steg (14, 15) begrenzt. Anschlag und Steg (22 und 14) sowie (21 und 15) bilden jeweils eine ebene Anlagefläche gegen die sich die Membran je nach Situation (Therapiebetrieb oder Therapieunterbrechung) anlegen kann.

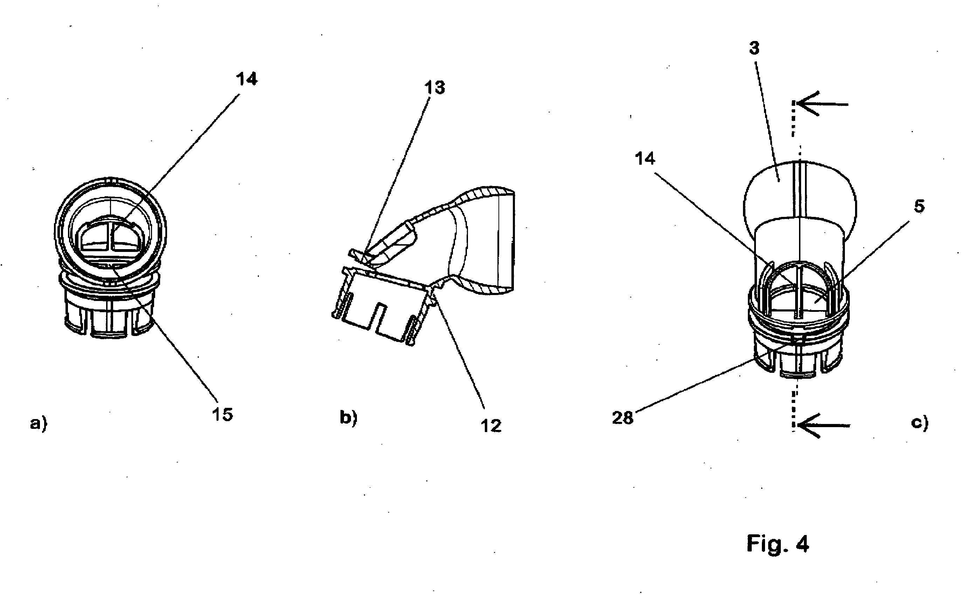

In den seitlichen Schnittdarstellung der Figuren 3 und 4 sind die Stege (14 und 15) teilweise nicht zu sehen, da der Schnittverlauf außerhalb der Mittelachse, direkt hinter den Stegen erfolgt, da sonst das sichtbare Material der Stege die Öffnungen verdecken würde.

Der Anschlag (21) ist wie der Steg (15) direkt im Flowkanal (11) des Anschlussstutzens (3) angeordnet und begrenzt den Membranweg (27), wenn eine Therapieunterbrechung vorliegt und kein Luftstrom in Richtung zum Patienten erfolgt. Der Patient atmet dann durch den stutzen und das Atemgas drückt die Membran gegen den Anschlag (21) und den Steg (15), die somit dem Luftweg in Richtung Schlauch zumindest teilweise verschließen. Der Anschlag (22), der zusammen mit dem Steg (14) an der Atmosphärenöffnung (5) angeordnet ist, begrenzt den Membranweg (27) derart, dass die Membran (7) die Atmosphärenöffnung (5) im Therapiebetrieb verschließen kann.

Die Drehhülse (2) weist einen Schlauchkonus (18), mit einem ersten Außendurchmesser, auf, auf welchen der Beatmungsschlauch geschoben werden kann. Drehhülse (2) weist einen zweiten Außendurchmesser im Bereich der Bedienfläche (20) auf. Der zweiten Außendurchmesser ist größer als der erste Außendurchmesser. Der zweite Außendurchmesser ist bevorzugt identisch zu dem Außendurchmesser des Anschlussstutzens, im Bereich der Kontaktfläche zur Drehhülse (2), gewählt. Die Drehhülse (2) weist innenseitig, in dem Abschnitt des zweiten Außendurchmessers, Verbindungselemente (19, Hinterschnitt) zur Verbindung mit dem Stutzen auf. Innenseitig bildet die Drehhülse einen umlaufenden Hinterschnitt (19) aus, der beispielsweise einen abgerundeten Übergang des Innendurchmessers von einem kleineren zu einem größeren Innendurchmesser darstellt. Der Anschlussstutzen bildet an seinem, der Drehhülse (2) zugewandten, Abschnitt korrespondierende Verbindungselemente (16, 17) aus. Der Anschlussstutzen weist hier Ausnehmungen (17a) auf, die Federlaschen (17b) freigeben. Die Federlaschen (17b) weisen an ihrem Ende leichte Verdickungen (16, Fasen) auf, die in dem Hinterschnitt (19) einrasten.

In Figur 4a ist die Lage der Stege (14 und 15) in dem Stutzen zu erkennen.

In Figur 4b sieht man den Stutzen im Querschnitt von der Seite. Die Nut (12) weist hier beispielsweise ein rechteckiges Profil auf und ist oberhalb der Öffnung (13) breiter ausgeführt als die Öffnung. Die Öffnung (13) verjüngt sich konisch.

In Figur 4c sieht man die Atmosphärenöffnung (5) und den drunter liegenden Steg (14). Benachbart zur Membranöffnung (13) ist die Führungsaufnahme (28) für den Membranring zu erkennen.

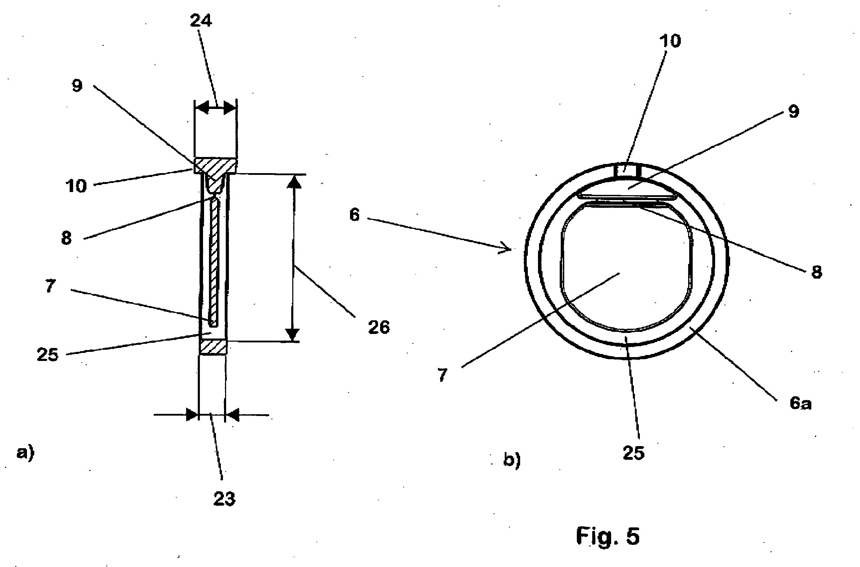

Fig.5a zeigt: Der Innendurchmesser (26) des Ringes ist kleiner als der Außendurchmesser des Anschlussstutzen (3) im Bereich der Membranöffnung (13), beispielsweise im Bereich der Nut (12), ausgebildet. Dadurch wird erreicht, dass der Ring (6) spielfrei montiert ist und sich durch die Aufweitung selbst zentriert. Die Breite (23, 24) des Ringes (6a) ist zumindest abschnittsweise größer als die Breite der Nut (12). Dadurch wird eine umlaufende Abdichtung des Ringes (6a) zum Anschlussstutzen (3) gewährleistet. Die Breite (24) des Ringes (6a) ist insbesondere im dem Bereich, der der Öffnung (13) anliegt, größer als die Breite der Nut (12), um die Öffnung dicht abzuschließen. Die Breite (23) kann kleiner sein als die Breite (24).

Fig. 5b zeigt: Die Membran (7) ist über ein Scharnier (8) mit dem Membranring (6) verbunden, wobei zwischen dem Scharnier (8) und dem Ring (6a) noch ein Dichtbereich (9) angeordnet ist. Dieser Dichtbereich (9) dient zum Verschließen der Membranöffnung (13) im Anschlussstutzen (3). Der Dichtbereich (9) ist konisch nach innen verjüngend ausgeführt und fügt sich bündig in die Membranöffnung (13) im Anschlussstutzen (3) ein.

Die Membran (7) weist eine Stärke bevorzugt von 0,5 mm bis 1,0 mm. Das Scharnier (8) weist eine bevorzugte Stärke / Dicke von 0,2 mm bis 0,5 mm auf, ist aber in jedem Fall dünner als die Membran (7). Die Breite des Scharniers (7) kann im Bereich zwischen 4,0 mm und 10,0 mm liegen und eine Länge von 0,2 mm bis 1,0 mm aufweisen. Figur 5 zeigt zudem, dass zwischen der Membran und dem Innendurchmesser (26) des Ringes ein Spalt (25) verbleibt, um ein freies Schwingen der Membran zu ermöglichen.

Als Material für den Membranring (6) ist ein Silikon oder ein Thermoplastisches Elastomere (TPE) mit einer shore-Härte im Bereich von 30 bis 50 Shore A, bevorzugt 40 Shore A, gewählt. Der Vorteil des innenliegenden Notfallausatemventils ist, dass kein zusätzliches äußeres Bauteil, welches verloren gehen kann, benötigt wird und dass keine zusätzliche äußere Abdichtung z.B. einer Einschubtasche oder Membrankassette erforderlich ist. Die Membran ist bevorzugt einteilig mit dem Membranring (6) ausgeführt und wird im Spritzgussverfahren hergestellt.

### Bezugsziffern:

- 1: Beatmungsschlauch (nicht dargestellt)
- 2: Drehhülse
- 3: Anschlussstutzen mit Kugelgelenk
- 4: Beatmungsmaske (nicht dargestellt)
- 5: Atmosphärenöffnung
- 6: Membranring
- 6a: Ring
- 7: Membran
- 8: Scharnier
- 9: Dichtbereich
- 10: Führungssteg
- 11: Flowkanal
- 12: Nut
- 13: Membranöffnung
- 14: Steg für Membrananschlag außen
- 15: Steg für Membrananschlag innen im Flowkanal
- 16: Fase
- 17a: Ausnehmung
- 17b: Federlasche
- 18: Schlauchkonus
- 19: Hinterschnitt
- 20: Bedienfläche
- 21: Anschlag
- 22: Anschlag
- 23: Breite 1
- 24: Breite 2
- 25: Spalt
- 26: Innendurchmesser
- 27: Membranweg
- 28: Führungsaufnahme / Verdrehsicherung

## Patentansprüche

1. Anschlussstutzen (3) für die Beatmung mit einer Öffnung die zum Beatmungsschlauch (1) weist und einer Öffnung die zum Patienteninterface (4) angeordnet ist und einer Atmosphärenöffnung (5) die von innen durch eine Membran (7) zumindest teilweise verschließbar ist **dadurch gekennzeichnet, dass** der Anschlussstutzen (3) eine seitliche Membranöffnung (13), für die Aufnahme der Membran (7) in den Stutzen, aufweist und die Membran (7) über einen Membranring (6), der den Anschlussstutzen (3) in vollem Umfang umschließt, gehalten ist.

2. Anschlussstutzen (3) nach Anspruch 1 **dadurch gekennzeichnet, dass** der Anschlussstutzen im Außenumfang zumindest abschnittsweise eine Nut (12) für den Membranring (6) aufweist.

3. Anschlussstutzen (3) nach Anspruch 1 oder 2 **dadurch gekennzeichnet, dass** ein Führungssteg (10) am Membranring (6) sowie eine korrespondierende Führungsaufnahme (28) im Anschlussstutzen (3) die Einbauposition von Membranring (6) und Membran (7) festlegen.

4. Anschlussstutzen (3) nach zumindest einem der vorhergehenden Ansprüche **dadurch gekennzeichnet, dass** der Membranweg (27) im Anschlussstutzen (3) durch zumindest einen Anschlag (21, 22) und zumindest einen Steg (14, 15) begrenzt ist und Anschlag und Steg jeweils eine Anlagefläche für die Membran bilden.

5. Anschlussstutzen (3) nach zumindest einem der vorhergehenden Ansprüche **dadurch gekennzeichnet, dass** der Anschlussstutzen (3) Verbindungselemente (17a, 17b) zur Verbindung mit einer Drehhülse (2) aufweist.

6. Anschlussstutzen (3) nach zumindest einem der vorhergehenden Ansprüche **dadurch gekennzeichnet, dass** der Anschlussstutzen (3) zumindest abschnittsweise einen Außendurchmesser aufweist, der dem Außendurchmesser des Membranringes (6) entspricht.

7. Anschlussstutzen (3) nach zumindest einem der vorhergehenden Ansprüche **dadurch gekennzeichnet, dass** der Anschlussstutzen (3) zumindest im Bereich der Membranöffnung (13) einen Außendurchmesser aufweist, der größer ist als der Innendurchmesser des Membranringes (6).

8. Anschlussstutzen (3) nach Anspruch 2, **dadurch gekennzeichnet, dass** die Breite (23, 24) des Membranringes (6) zumindest abschnittsweise größer als die Breite der Nut (12) ist.

9. Anschlussstutzen (3) nach zumindest einem der vorhergehenden Ansprüche **dadurch gekennzeichnet, dass** die Membranöffnung (13) konisch nach innen verjüngend ausgeführt ist.

10. Anschlussstutzen (3) nach zumindest einem der vorhergehenden Ansprüche **dadurch gekennzeichnet, dass** die Membran (7) über ein Scharnier (8) mit dem Membranring (6) verbunden ist.

11. Anschlussstutzen (3) nach Anspruch 10 **dadurch gekennzeichnet, dass** der Membranring (6) einen Ring (6a) aufweist und zwischen dem Scharnier (8) und dem Ring (6a) noch ein Dichtbereich (9) angeordnet ist, der zum Verschließen der Membranöffnung (13) im Anschlussstutzen (3) dient.

12. Anschlussstutzen (3) nach zumindest einem der vorhergehenden Ansprüche **dadurch gekennzeichnet, dass** der Membranring (6) mit der Membran (7) einteilig aus Silikon oder einem anderen Elastomer gefertigt ist.

13. Anschlussstutzen (3) nach einem der Ansprüche 10 und 11 oder nach Anspruch 12 wenn abhängig vom Anspruch 10 oder 11 **dadurch gekennzeichnet, dass** die Membran (7) bevorzugt eine Stärke von 0,4 mm bis 1,0 mm aufweist und das Scharnier (8) dünner als die Membran (7)ist.

14. Anschlussstutzen (3) nach zumindest einem der vorhergehenden Ansprüche **dadurch gekennzeichnet, dass** zwischen der Membran (7) und dem Innendurchmesser (26) des Ringes (6a) ein Spalt (25) verbleibt, um ein freies Schwingen der Membran zu ermöglichen.

## Claims

1. A connection piece (3) for respiration comprising an opening pointing toward the breathing tube (1) and an opening arranged toward the patient interface (4) and an atmosphere opening (5) that can be closed from the inside at least in part by means of a membrane (7), **characterized in that** the connection piece (3) comprises a lateral membrane opening (13) for receiving the membrane (7) in the connection piece and the membrane (7) is held by means of a membrane ring (6) that fully encircles the connection piece (3).

2. The connection piece (3) according to claim 1, **characterized in that** the connection piece comprises a groove (12) for the membrane ring (6) on at least part of the outer circumference.

3. The connection piece (3) according to claim 1 or 2, **characterized in that** a guide tab (10) on the membrane ring (6) and a corresponding guide receiving portion (28) in the connection piece (3) set the assembly position of the membrane ring (6) and membrane (7).

4. The connection piece (3) according to at least one of the preceding claims, **characterized in that** the membrane travel (27) within the connection piece (3) is limited by means of at least one stop (21, 22) and at least one rib (14,15) and the stop and rib in each case form a contact surface for the membrane.

5. The connection piece (3) according to at least one of the preceding claims, **characterized in that** the connection piece (3) comprises coupling elements (17a, 17b) for coupling to a rotary sleeve (2).

6. The connection piece (3) according to at least one of the preceding claims, **characterized in that** the connection piece (3) has an outer diameter that corresponds to the outer diameter of the membrane ring (6) at least in portions.

7. The connection piece (3) according to at least one of the preceding claims, **characterized in that** the connection piece (3) has an outer diameter at least in the region of the membrane opening (13) that is greater than the inner diameter of the membrane ring (6).

8. The connection piece (3) according to claim 2, **characterized in that** the width (23, 24) of the membrane ring (6) is greater than the width of the groove (12) at least in portions.

9. The connection piece (3) according to at least one of the preceding claims, **characterized in that** the membrane opening (13) is conical and tapers inward.

10. The connection piece (3) according to at least one of the preceding claims, **characterized in that** the membrane (7) is coupled to the membrane ring (6) by means of a hinge (8).

11. The connection piece (3) according to claim 10, **characterized in that** the membrane ring (6) comprises a ring (6a) and a sealing portion (9) for plugging the membrane opening (13) in the connection piece (3) is arranged between the hinge (8) and the ring (6a).

12. The connection piece (3) according to at least one of the preceding claims, **characterized in that** the membrane ring (6) is manufactured integrally with the membrane (7) from silicone or another elastomer.

13. The connection piece (3) according to at least one of the preceding claims, **characterized in that** the membrane (7) preferably has a thickness of from 0.4 mm to 1.0 mm and the hinge (8) is thinner than the membrane (7).

14. The connection piece (3) according to any one of claims 10 or 11 or according to claim 12 if dependent on claim 10 or 11, **characterized in that** a gap (25) is left between the membrane (7) and the inner diameter (26) of the ring (6a) so as to allow free swinging of the membrane.

## Revendications

1. Tubulure de raccordement (3) servant à la respiration artificielle, dotée d'une ouverture qui est orientée vers le tube respiratoire (1), d'une ouverture qui est disposée par rapport à l'interface patient (4) et d'une ouverture sur l'atmosphère ambiante (5) qui peut être obturée au moins en partie de l'intérieur par une membrane (7), **caractérisée en ce que** la tubulure de raccordement (3) présente une ouverture de membrane latérale (13) pour loger la membrane (7) dans la tubulure et que la membrane (7) est maintenue au moyen d'une bague de membrane (6), qui enserre la tubulure de raccordement (3) sur tout son pourtour.

2. Tubulure de raccordement (3) selon la revendication 1 **caractérisée en ce que** la tubulure de raccordement présente au moins par endroits sur son pourtour extérieur une rainure (12) destinée à la bague de membrane (6).

3. Tubulure de raccordement (3) selon la revendication 1 ou 2 **caractérisée en ce qu'**un ergot de guidage (10) prévu sur la bague de membrane (6) ainsi qu'un logement de guidage correspondant (28) aménagé dans la tubulure de raccordement (3) définissent la position de montage de la bague de membrane (6) et de la membrane (7) .

4. Tubulure de raccordement (3) selon l'une au moins des revendications précédentes, **caractérisée en ce que** le trajet de la membrane (27) dans la tubulure de raccordement (3) est limité par au moins une butée (21, 22) et par au moins une entretoise (14, 15) et que la butée et l'entretoise forment respectivement une surface d'appui de la membrane.

5. Tubulure de raccordement (3) selon l'une au moins des revendications précédentes, **caractérisée en ce que** la tubulure de raccordement (3) présente des éléments d'assemblage (17a, 17b) à raccorder à une douille rotative (2).

6. Tubulure de raccordement (3) selon l'une au moins des revendications précédentes, **caractérisée en ce que** la tubulure de raccordement (3) présente au moins par endroits un diamètre extérieur, qui équivaut au diamètre extérieur de la bague de membrane (6).

7. Tubulure de raccordement (3) selon l'une au moins des revendications précédentes, **caractérisée en ce que** la tubulure de raccordement (3) présente au moins dans la zone de l'ouverture de la membrane (13) un diamètre extérieur, qui est supérieur au diamètre intérieur de la bague de membrane (6).

8. Tubulure de raccordement (3) selon la revendication 2 **caractérisée en ce que** la largeur (23, 24) de la bague de membrane (6) est plus grande au moins par endroits que la largeur de la rainure (12).

9. Tubulure de raccordement (3) selon l'une au moins des revendications précédentes, **caractérisée en ce que** l'ouverture de la membrane (13) est réalisée sous une forme conique se rétrécissant vers l'intérieur.

10. Tubulure de raccordement (3) selon l'une au moins des revendications précédentes, **caractérisée en ce que** la membrane (7) est reliée à la bague de membrane (6) par une charnière (8).

11. Tubulure de raccordement (3) selon la revendication 10 **caractérisée en ce que** la bague de membrane (6) présente une bague (6a) et qu'une zone d'étanchéité (9) est encore disposée entre la charnière (8) et la bague (6a), qui sert à obturer l'ouverture de la membrane (13) dans la tubulure de raccordement (3).

12. Tubulure de raccordement (3) selon l'une au moins des revendications précédentes, **caractérisée en ce que** la bague de membrane (6) est fabriquée d'un seul tenant avec la membrane (7) à base de silicone ou d'un autre élastomère.

13. Tubulure de raccordement (3) selon l'une au moins des revendications précédentes, **caractérisée en ce que** la membrane (7) présente de préférence une épaisseur de 0,4 mm à 1,0 mm et que la charnière (8) est plus mince que la membrane (7).

14. Tubulure de raccordement (3) selon l'une des revendications 10 et 11 ou selon la revendication 12, lorsqu'elle est dépendante de la revendication 10 ou 11, **caractérisée en ce qu'**un interstice (25) subsiste entre la membrane (7) et le diamètre intérieur (26) de la bague (6a) pour permettre à la membrane de vibrer librement.
